# EUROPEAN PATENT APPLICATION

(11) **EP 3 378 386 A1**
(43) Date of publication of application: **26.09.2018**
(21) Application number: 17162313.5
(22) Date of filing: 22.03.2017
(51) Int. Cl.: A61B 5/024, G06F 19/00

(54) **METHOD AND APPARATUS FOR DETERMINING A HEALTH STATUS OF AN INFANT**

(71) Applicant: Koninklijke Philips N.V., 5656 AE Eindhoven (NL)
(72) Inventor: ATALLAH, Louis Nicolas, 5656 AE Eindhoven (NL); Werth, Jan, 5656 AE Eindhoven (NL)
(74) Representative: de Haan, Poul Erik

(57) **Abstract**

There is provided an apparatus comprising a control unit and a method of operating the apparatus to determine a health status of an infant. The method comprises acquiring contextual information associated with the infant (302) and acquiring at least one heart rate variability signal from the infant (304). The at least one heart rate variability signal acquired from the infant is processed to determine a heart rate variability feature (306) and the determined heart rate variability feature is assigned to a class according to the acquired contextual information (308). The determined heart rate variability feature is compared to one or more corresponding heart rate variability features stored in the same class as the determined heart rate variability in a memory (310) and a health status of the infant is determined based on the comparison (312).

## Description

### TECHNICAL FIELD OF THE INVENTION

The invention relates to the field of infant care and, in particular, to a method and apparatus for determining a health status of an infant.

### BACKGROUND TO THE INVENTION

Heart rate variability (HRV) features have, in the past, been used to assess comfort or stress levels of infants. However, heart rate variability features are affected by many external factors such as positioning, sleep state and interventions. The variety of factors that can affect heart rate variability features make it difficult for the features to be reliably used for stress assessment for infants and can lead to unreliable determinations of stress levels. Due to the potentially severe influence that external factors can have on heart rate variability features, heart rate variability features are still not widely used in neonatal intensive care units (NICUs).

Other systems have been developed to determine the stress levels of an infant based on features aside from heart rate variability. For example, US 2012/0157757 A1 discloses a system that analyses both an infant motion intensity as well as changes in physiological parameters of the infant in evaluating a stress level of the infant. The infant is determined to be experiencing increased stress levels where, for example, a heart rate graph and an SpO2 graph fail to return to their respective levels during a recovery phase following a clinical intervention.

However, while alternative methods for stress level determination exist, heart rate variability features can provide particularly valuable information on the health status of an infant. For example, heart rate variability features can be used to non-invasively assess the autonomic function of infants (such as the balance between the sympathetic and parasympathetic nervous system of infants). It would thus be useful to provide a more reliable assessment of the health of infants using heart rate variability features.

There is thus a need for an improved method and apparatus for determining a health status of an infant.

### SUMMARY OF THE INVENTION

As noted above, the limitation with existing approaches is that heart rate variability features are severely influenced by external factors and are thus unreliable in infant health status determination. It would thus be beneficial to have an improved method and apparatus for determining a health status of an infant, which overcomes these existing problems.

Therefore, according to a first aspect of the invention, there is provided a method of operating an apparatus comprising a control unit to determine a health status of an infant. The method comprises acquiring contextual information associated with the infant, acquiring at least one heart rate variability signal from the infant, processing the at least one heart rate variability signal acquired from the infant to determine a heart rate variability feature, assigning the determined heart rate variability feature to a class according to the acquired contextual information, comparing the determined heart rate variability feature to one or more corresponding heart rate variability features stored in the same class as the determined heart rate variability in a memory, and determining a health status of the infant based on the comparison.

In some embodiments, comparing may comprise determining a ratio of the determined heart rate variability feature to one of the corresponding heart rate variability features stored in the same class as the determined heart rate variability in the memory.

In some embodiments, determining a health status of the infant may comprise determining an abnormal health status of the infant when the determined heart rate variability feature differs by more than a predefined threshold from the one or more corresponding heart rate variability features stored in the same class as the determined heart rate variability in the memory.

In some embodiments, the method may further comprise plotting the determined heart rate variability feature against the acquired contextual information over time and comparing may comprise comparing the plot of the determined heart rate variability feature against the acquired contextual information over time with a plot of the one or more corresponding heart rate variability features against corresponding contextual information over time.

In some embodiments, the method may further comprise selecting a baseline for the determined heart rate variability feature based on the acquired contextual information and normalizing the determined heart rate variability feature with the baseline.

In some embodiments, selecting a baseline may comprise plotting the at least one heart rate variability signal acquired from the infant against the acquired contextual information over time, detecting a time period in which the acquired contextual information is below a predefined threshold, and selecting a baseline for the determined heart rate variability feature from corresponding heart rate variability features in the detected time period. In some embodiments, comparing may comprise comparing the normalized heart rate variability feature to one or more corresponding heart rate variability features stored in the same class as the normalized heart rate variability in a memory.

In some embodiments, the determined heart rate variability feature may comprise any one or more of: an increase in heart rate variability signal, a decrease in heart rate variability signal, a standard deviation of a time interval between heart beats in the heart rate variability signal, a square root of mean squares of successive differences between adjacent heart beats in the heart rate variability signal, a number of interval differences of successive heart beat intervals in the heart rate variability signal that are greater than a threshold interval, a power in the frequency domain of the heart rate variability signal, a cardiac autonomic balance of the heart rate variability signal, a standard deviation perpendicular to a line of identity in a Poincaré plot of the heart rate variability signal, and a standard deviation along a line of identity in a Poincaré plot of the heart rate variability signal.

In some embodiments, the contextual information may comprise information that has an influence on the heart rate variability of the infant. In some embodiments, the contextual information may comprise information on any one or more of: an age of the infant, a size of the infant, a developmental stage of the infant, a feeding status of the infant, a sleep state of the infant, a sleep or wake level of the infant, a lying position of the infant, a movement of the infant, an activity level of the infant, a heart rate of the infant, a respiration rate of the infant, a body temperature of the infant, a skin color of the infant, a facial expression of the infant, an intervention with the infant, an ambient temperature in the environment of the infant, and an irregularity with equipment associated with the infant.

According to a second aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to a third aspect of the invention, there is provided an apparatus for determining a health status of an infant. The apparatus comprises a control unit configured to acquire contextual information associated with the infant, acquire at least one heart rate variability signal from the infant, process the at least one heart rate variability signal acquired from the infant to determine a heart rate variability feature, assign the determined heart rate variability feature to a class according to the acquired contextual information, compare the determined heart rate variability feature to one or more corresponding heart rate variability features stored in the same class as the determined heart rate variability in a memory and determine a health status of the infant based on the comparison.

In some embodiments, the control unit may be configured to acquire the at least one heart rate variability signal from the infant by controlling at least one heart rate variability sensor to acquire the at least one heart rate variability signal from the infant. In some embodiments, the control unit may be configured to acquire the contextual information associated with the infant from a memory or by controlling at least one contextual information sensor in the environment of the infant to acquire the contextual information.

In some embodiments, the at least one heart rate variability sensor may comprise any one or more of: a camera, a motion sensor, a laser Doppler sensor, a Doppler radar sensor, a needle patch sensor, an electrocardiography (ECG) sensor, a photoplethysmography (PPG) sensor, a ballistocardiography (BCG) sensor, an instrumented catheter sensor, and an instrumented feeding tube sensor. In some embodiments, the contextual information sensor may comprise any one or more of: a camera directed at the infant, an audio sensor in the environment of the infant, a motion sensor in the environment of the infant, a pressure sensor in a surface on which the infant lies, a temperature sensor in the environment of the infant, and a physiological characteristic sensor.

In some embodiments, the control unit may be further configured to control a user interface to output any one or more of the determined heart rate variability feature and the determined health status of the infant.

According to the aspects and embodiments described above, the limitations of existing techniques are addressed. In particular, according to the above-described aspects and embodiments, it is possible to detect context and essentially calibrate heart rate variability features based on this detected context. In this way, a more accurate interpretation of heart rate variability features can be achieved, thereby leading to a more reliable determination of a health status of an infant. Moreover, a more standardized interpretation of heart rate variability features as indicators of a health status is provided. There is thus provided a method and apparatus for determining a health status of an infant, which overcomes the existing problems.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of an apparatus according to an embodiment;
Fig. 2 is an illustration of the apparatus in use according to an embodiment;
Fig. 3 is a flow chart illustrating a method according to an embodiment;
Fig. 4 is a graphical illustration of a electrocardiography (ECG) signal acquired from an infant according to an embodiment; and
Fig. 5 is a graphical illustration of a heart rate variability feature for an infant according to an embodiment.

### DETAILED DESCRIPTION OF EMBODIMENTS

As noted above, the invention provides a method and apparatus for determining a health status of an infant, which overcomes the existing problems.

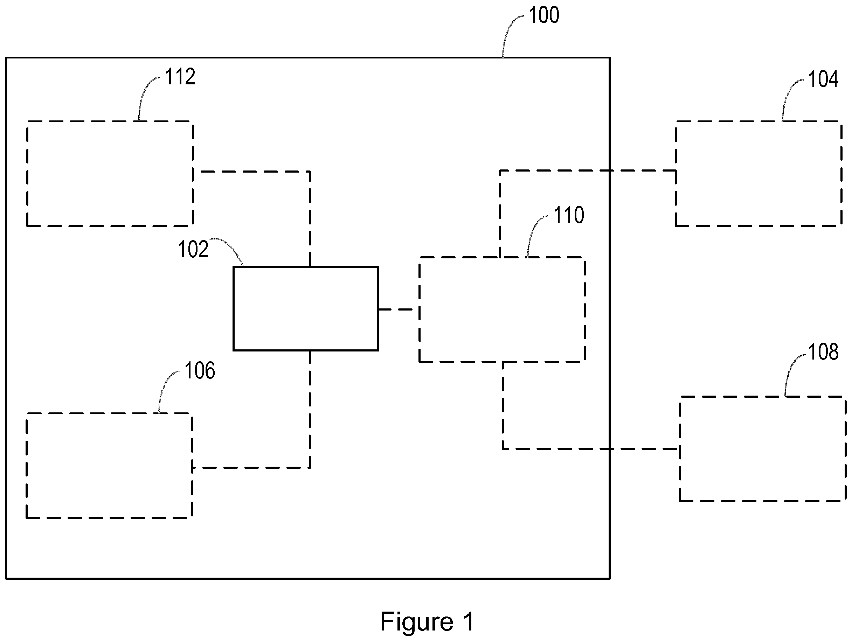

Fig. 1 shows a block diagram of an apparatus 100 according to an embodiment that can be used for determining a health status of an infant. An infant can, for example, be a neonate (such as a pre-term infant, a full-term infant or a post-term infant) or a child.

With reference to Fig. 1, the apparatus 100 comprises a control unit 102 that controls the operation of the apparatus 100 and that can implement the method described herein. The control unit 102 can comprise one or more processors, processing units, multicore processors or modules that are configured or programmed to control the apparatus 100 in the manner described herein. In particular implementations, the control unit 102 can comprise a plurality of software and/or hardware modules that are each configured to perform, or are for performing, individual or multiple steps of the method according to embodiments of the invention.

Briefly, the control unit 102 is configured to acquire contextual information associated with an infant, acquire at least one heart rate variability signal from the infant, process the at least one heart rate variability signal acquired from the infant to determine a heart rate variability feature, assign the determined heart rate variability feature to a class according to the acquired contextual information, compare the determined heart rate variability feature to one or more corresponding heart rate variability features stored in the same class as the determined heart rate variability in a memory, and determine a health status of the infant based on the comparison.

As mentioned above, the control unit 102 is configured to acquire at least one heart rate variability signal from the infant. A heart rate variability signal conveys (or represents) variations in a heart rate of the infant over time. In some embodiments, the control unit 102 may be configured to acquire the at least one heart rate variability signal from the infant by controlling at least one heart rate variability sensor 104 to acquire the at least one heart rate variability signal from the infant. Examples of the at least one heart rate variability sensor 104 include, but are not limited to, any one or more of a camera, a motion sensor (such as an accelerometer), a laser Doppler sensor, a Doppler radar sensor, a needle patch sensor, an electrocardiography (ECG) sensor, a photoplethysmography (PPG) sensor, a ballistocardiography (BCG) sensor, an instrumented catheter sensor, an instrumented feeding tube sensor, or any other heart rate variability sensor, or any combination of heart rate variability sensors.

As mentioned above, the control unit 102 is also configured to acquire contextual information associated with the infant. In some embodiments, the control unit 102 may be configured to acquire the contextual information associated with the infant from a memory 106. As illustrated in Fig. 1, according to some embodiments, the apparatus 100 may comprise a memory 106. A memory 106 of the apparatus 100 may be configured to store program code that can be executed by the control unit 102 to perform the method described herein. Alternatively or in addition to the apparatus 100 comprising a memory 106, one or more memories 106 may be external to (i.e. separate to or remote from) the apparatus 100. For example, one or more memories 106 may be part of another device or The Cloud.

A memory 106 can be used to store information, data, signals and measurements acquired or made by the control unit 102 of the apparatus 100 or from any components, units, interfaces, sensors, memories, or devices that are external to the apparatus 100. The control unit 102 may be configured to control a memory 106 to store information, data, signals and measurements resulting from the method disclosed herein. For example, in some embodiments, contextual information associated with the infant may be stored in the memory 106. In these embodiments, the control unit 102 may be configured to acquire the contextual information associated with the infant from the memory 106. A memory 106 may store, for example, the one or more corresponding heart rate variability features, the determined health status of the infant, or any other information, or any combination of information, resulting from the method disclosed herein. The one or more corresponding heart rate variability features may, for example, be stored in the form of a histogram of infants, a class distribution of infants, and/or look-up table of infants.

Alternatively or in addition to acquiring contextual information from a memory 106, the control unit 102 may be configured to acquire contextual information associated with the infant by controlling at least one contextual information sensor 108 in the environment of the infant to acquire the contextual information. The location of the at least one contextual information sensor 108 in the environment of the infant may be any location at which the at least one contextual information sensor 108 can acquire contextual information associated with the infant. Examples of the contextual information sensor 108 include, but are not limited to, any one or more of a camera directed at the infant, an audio sensor in the environment of the infant, a motion sensor in the environment of the infant, a pressure sensor in a surface on which the infant lies, a temperature sensor in the environment of the infant, a physiological characteristic sensor, or any other contextual information sensor, or any combination of contextual information sensors.

A camera directed at the infant may, for example, be a camera attached to a neonatal incubator enclosing the infant, a camera directed at a bed of the infant, a camera placed at a location in proximity of the infant, or a camera at any other location where the camera is directed at the infant. In some embodiments, contextual information may be acquired from one or more cameras. In the case of more than one camera, contextual information may be acquired from cameras at different locations in the environment of the infant, cameras at the same location in the environment of the infant, or a combination of both. A camera can comprise any camera suitable to acquire contextual information associated with the infant or, more specifically, one or more images associated with the infant (which may comprise images associated with the infant themselves and/or images associated with the environment of the infant). Examples of a camera include, but are not limited to, a thermal camera, an infra-red (IR) camera, or any other camera, or any combination of cameras, suitable for acquiring contextual information associated with the infant.

An audio sensor in the environment of the infant may, for example, be an audio sensor attached to the inside of a neonatal incubator enclosing the infant, an audio sensor placed in proximity of the infant (which, in the case of a neonate, may be inside or outside a neonatal incubator enclosing the neonate), or an audio sensor at any other location in the environment of the infant. In some embodiments, contextual information may be acquired from one or more audio sensors. In the case of more than one audio sensor, contextual information may be acquired from audio sensors at different locations in the environment of the infant, audio sensors at the same location in the environment of the infant, or a combination of both. An audio sensor can comprise any audio sensor suitable to acquire contextual information associated with the infant or, more specifically, audio associated with the infant (which may be audio associated with the infant themselves and/or audio associated with the environment of the infant). Examples of an audio sensor include, but are not limited to, a microphone, or any other audio sensor, or any combination of audio sensors, suitable for acquiring contextual information associated with the infant.

A motion sensor in the environment of the infant may, for example, be a motion sensor attached to the infant, a motion sensor located on or in a surface on which the infant lies (such as a surface of a support structure of a neonatal incubator, a surface of a mattress on a bed of the infant, or any other surface on which the infant lies), a motion sensor directed at the infant, or a motion sensor at any other location in the environment of the infant. In some embodiments, contextual information may be acquired from one or more motion sensors. In the case of more than one motion sensor, contextual information may be acquired from motion sensors at different locations in the environment of the infant, motion sensors at the same location in the environment of the infant, or a combination of both. A motion sensor can comprise any motion sensor suitable to acquire contextual information associated with the infant or, more specifically, motion information associated with the infant (which may be motion information associated with the infant themselves and/or motion information associated with the environment of the infant). Examples of a motion sensor include, but are not limited to, an accelerometer, a gyroscope, a magnetometer, a camera (such as any of those mentioned earlier), or any other motion sensor, or any combination of motion sensors, suitable for acquiring contextual information associated with the infant.

A pressure sensor in a surface on which the infant lies may, for example, be a pressure sensor in a surface of a support structure of a neonatal incubator, a surface of a mattress on a bed of the infant, or any other surface on which the infant lies. In some embodiments, contextual information may be acquired from one or more pressure sensors. In the case of more than one pressure sensor, contextual information may be acquired from pressure sensors at different locations in the surface on which the infant lies, pressure sensors at the same location in the surface on which the infant lies, or a combination of both. A pressure sensor can comprise any pressure sensor suitable to acquire contextual information associated with the infant or, more specifically, pressure or positional information associated with the infant (which may be pressure or positional information associated with the infant themselves). Examples of a pressure sensor include, but are not limited to, pressure sensitive elements (such as pressure sensitive non-contacting electrodes), capacitive tactile sensors, or any other pressure sensor, or any combination of pressure sensors, suitable for acquiring contextual information associated with the infant.

A temperature sensor in the environment of the infant may, for example, be a temperature sensor attached to the infant, a temperature sensor attached to the inside of a neonatal incubator enclosing the infant, a temperature sensor placed in proximity of the infant (which, in the case of a neonate, may be inside or outside a neonatal incubator enclosing the neonate), or a temperature sensor at any other location in the environment of the infant. In some embodiments, contextual information may be acquired from one or more temperature sensors. In the case of more than one temperature sensor, contextual information may be acquired from temperature sensors at different locations in the environment of the infant, temperature sensors at the same location in the environment of the infant, or a combination of both. A temperature sensor can comprise any temperature sensor suitable to acquire contextual information associated with the infant or, more specifically, temperature information associated with the infant (which may be temperature information associated with the infant themselves and/or temperature information associated with the environment of the infant). Examples of a temperature sensor include, but are not limited to, a thermometer, a temperature probe (such as a temperature probe to be placed in a diaper of the infant) or any other temperature sensor, or any combination of temperature sensors, suitable for acquiring contextual information associated with the infant.

A physiological characteristic sensor in the environment of the infant may, for example, be a physiological characteristic sensor attached to the infant, a physiological characteristic sensor attached to the inside of a neonatal incubator enclosing the infant, a physiological characteristic sensor placed in proximity of the infant (which, in the case of a neonate, may be inside or outside a neonatal incubator enclosing the neonate), a physiological characteristic sensor in a surface on which the infant lies (for example, a surface of a support structure of a neonatal incubator, a surface of a mattress on a bed of the infant, or any other surface on which the infant lies), or a physiological characteristic sensor at any other location in the environment of the infant. In some embodiments, contextual information may be acquired from one or more physiological characteristic sensors. In the case of more than one physiological characteristic sensor, contextual information may be acquired from physiological characteristic sensors at different locations in the environment of the infant, physiological characteristic sensors at the same location in the environment of the infant, or a combination of both.

A physiological characteristic sensor can comprise any physiological characteristic sensor suitable to acquire contextual information associated with the infant or, more specifically, physiological characteristic information of the infant. Examples of a physiological characteristic sensor include, but are not limited to, a heart rate sensor (such as an electrocardiography, ECG, sensor, a photoplethysmography, PPG, sensor, or any other heart rate sensor, or any combination of heart rate sensors), a respiration sensor (such as an air-flow sensor, or any other respiration sensor, or any combination of respiration sensors), a skin conductivity sensor (such as a galvanic skin response, GSR, sensor, or any other skin conductivity sensor, or any combination of skin conductivity sensors), a brain activity sensor (such as an electroencephalography, EEG, sensor, or any other brain activity sensor, or any combination of brain activity sensors), a blood oxygen saturation (SpO2) sensor (such as a pulse oximeter, or any other blood oxygen saturation sensor, or any combination of blood oxygen saturation sensors), a muscle activity sensor (such as an electromyography, EMG, sensor, or any other muscle activity sensor, or any combination of muscle activity sensors), or any other physiological characteristic sensor, or any combination of physiological characteristic sensors, suitable for acquiring contextual information associated with the infant.

The contextual information associated with the infant can comprise any information that has an influence on (or that affects) the heart rate variability of the infant. Examples of the contextual information include, but are not limited to, any one or more of an age of the infant, a size of the infant, a developmental stage or maturation of the infant (for example, for a neonate, this may be pre-term such as less than or equal to 36 weeks gestational age (wGA), intermediate such as more than 36 wGA and less than 40 wGA, and full-term such as greater than or equal to 40 wGA), a feeding status of the infant, a sleep state of the infant (such as active sleep AS state and quiet sleep QS state), a sleep or wake level of the infant, a lying position of the infant (such as information indicative of the infant lying supine, prone, or on a side), a movement of the infant, an activity level (such as low, medium or high activity level) of the infant, a heart rate of the infant, a respiration rate of the infant (or breathing rhythm of the infant), a body temperature (or change in body temperature) of the infant, a skin color of the infant, a facial expression (such as an abnormal facial expression) of the infant, an intervention with the infant (such as an intervention by a medical professional, a care taker, a family member, or any other person), an ambient temperature (or change in ambient temperature) in the environment of the infant, an irregularity with equipment (such as a pulled cable or any other irregularity) associated with the infant, or any other contextual information, or any combination of contextual information associated with the infant.

An age of the infant, a size of the infant and a developmental stage of the infant are examples of contextual information associated with the infant that may be acquired from a memory 106 (which may comprise one or more memories 106 of the apparatus 100, one or more memories 106 external to the apparatus 100, or a combination thereof). A feeding status of the infant, a sleep state of the infant, a sleep or wake level of the infant, a lying position of the infant, a movement of the infant, an activity level of the infant, a heart rate of the infant, a respiration rate of the infant, a body temperature of the infant, a skin color of the infant, a facial expression of the infant, an intervention with the infant, an ambient temperature in the environment of the infant, and an irregularity with equipment associated with the infant are examples of contextual information associated with the infant that may be acquired from a contextual information sensor 108 in the environment of the infant.

For example, any one or more of a feeding status of the infant, a sleep state of the infant, a sleep or wake level of the infant, a skin color of the infant, a facial expression of the infant, and an intervention with the infant, may be acquired from a camera directed at the infant. A lying position of the infant may be acquired from a pressure sensor in a surface on which the infant lies. Any one or more of a movement of the infant and an activity level of the infant may be acquired from any one or more of from a motion sensor in the environment of the infant and an audio sensor in the environment of the infant. Any one or more of a heart rate of the infant, a respiration rate of the infant and a body temperature of the infant may be acquired from an appropriate physiological characteristic sensor in the environment of the infant. An ambient temperature in the environment of the infant may be acquired from a temperature sensor (such as a thermometer) in the environment of the infant. An irregularity with equipment associated with the infant may be acquired from the equipment itself.

As illustrated in Fig. 1, in some embodiments, the apparatus 100 may also comprise a communications interface (or circuitry) 110 for enabling the apparatus 100 to communicate with (or connect to) any components, interfaces, units, memories, sensors and devices that are internal or external to the apparatus 100. The communications interface 110 may communicate with any components, interfaces, units, sensors and devices wirelessly or via a wired connection. For example, in embodiments where the control unit 102 controls at least one heart rate variability sensor 104 to acquire at least one heart rate variability signal from the infant, the communications interface 110 may communicate with the heart rate variability sensor wirelessly or via a wired connection. Similarly, in embodiments where the control unit 102 controls at least one contextual information sensor 108 in the environment of the infant to acquire the contextual information, the communications interface 110 may communicate with the at least one contextual information sensor 108 wirelessly or via a wired connection.

According to some embodiments, the apparatus 100 may also comprise at least one user interface 112. Alternatively or in addition, a user interface 112 may be external to (i.e. separate to or remote from) the apparatus 100. For example, the user interface 112 may be part of another device, such as a mobile device, or a central station, which may be set up to remotely monitor the health status of a plurality of infants. A user interface 112 may be for use in providing a user (which may be a medical professional, a care taker, a family member, or any other user) with information resulting from the method according to the invention. The control unit 102 may be configured to control one or more user interfaces 112 to provide information resulting from the method according to the invention. For example, in some embodiments, the control unit 102 may be configured to control a user interface 112 to render (or output or provide) the determined heart rate variability feature, the determined health status of the infant, both the determined heart rate variability feature and the determined health status of the infant, or any other information, or any combination of information, resulting from the method disclosed herein. A user interface 112 may, alternatively or in addition, be configured to receive a user input. In other words, a user interface 112 may allow the user of the apparatus 100 to manually enter data, instructions, or information. The control unit 102 may be configured to acquire the user input from one or more user interfaces 112.

A user interface 112 may be any user interface that enables rendering (or outputting or providing) of information, data or signals to a user of the apparatus 100. Alternatively or in addition, a user interface 112 may be any user interface that enables a user of the apparatus 100 to provide a user input, interact with and/or control the apparatus 100. For example, the user interface 112 may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render (or output or provide) information, data or signals of the apparatus 100 may be the same user interface as that which enables the user to provide a user input, interact with and/or control the apparatus 100.

It will be appreciated that Fig. 1 only shows the components required to illustrate this aspect of the invention, and in a practical implementation the apparatus 100 may comprise additional components to those shown. For example, the apparatus 100 may comprise a battery or other power supply for powering the apparatus 100 or means for connecting the apparatus 100 to a mains power supply.

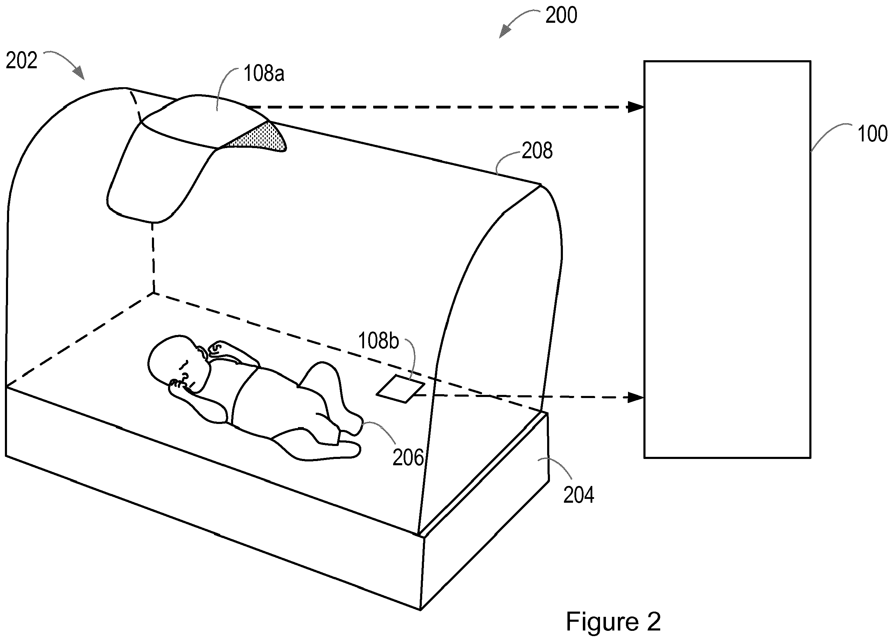

Fig. 2 is a block diagram of the apparatus 100 in use in an example system 200 according to an embodiment. In the illustrated example embodiment, the system 200 comprises the apparatus 100 (as described earlier) and a neonatal incubator 202. The neonatal incubator 202 comprises a support structure 204 configured to support an infant (specifically, a neonate in this illustrated example embodiment) 206 and an enclosure 208 configured to enclose (or contain) the neonate 206.
In the illustrated example embodiment of Fig. 2, the system 200 comprises a first contextual information sensor 108a and a second contextual information sensor 108b. The first contextual information sensor 108a is positioned over the top of the enclosure 208 of the neonatal incubator 202. The first contextual information sensor 108a can comprise, for example, one or more cameras directed at the neonate 206. The second contextual information sensor 108b is positioned inside the enclosure 208 of the neonatal incubator 202 on (or inside the surface of) the support structure 204. The second contextual information sensor 108b can comprise, for example, a temperature sensor configured to sense the temperature in the environment of the neonate 206.

In the illustrated example embodiment of Fig. 2, the control unit 102 of the apparatus 100 is configured to acquire the at least one heart rate variability signal from the neonate 206 by controlling at least one heart rate variability sensor (which is not shown in Fig. 2) to acquire the at least one heart rate variability signal and to acquire contextual information associated with the neonate 206 by controlling one or more of the first contextual information sensor 108a and the second contextual information sensor 108b to acquire the contextual information associated with the neonate 206.

Although examples have been provided above for the type of heart rate variability sensor, the type of contextual information sensor, and for the arrangement of those sensors, it will be understood that other types of sensor or combinations of sensors and other sensor arrangements are also possible.

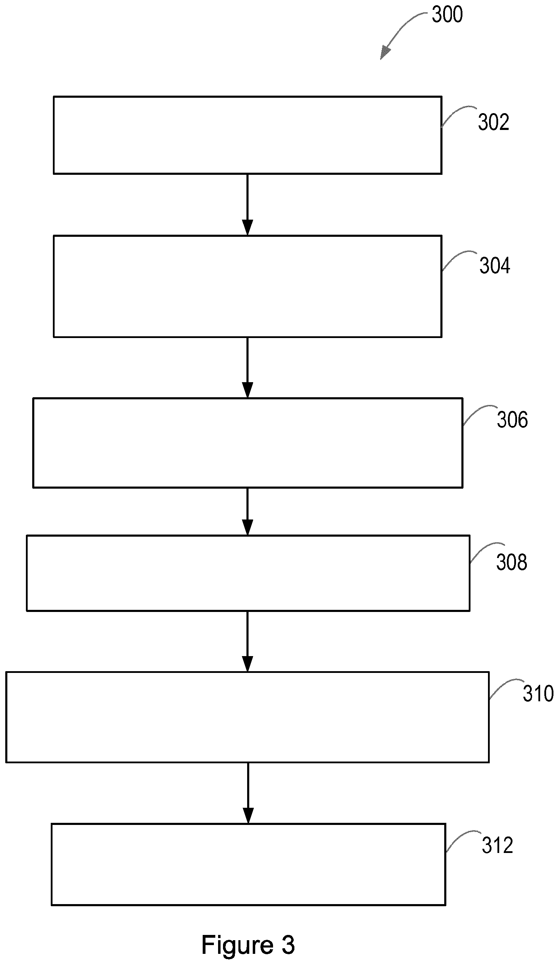

Fig. 3 illustrates a method 300 of operating an apparatus 100 comprising a control unit 102 to determine a health status of an infant according to an embodiment. As mentioned earlier, an infant can, for example, be a neonate (such as a pre-term infant, a full-term infant or a post-term infant) or a child. The illustrated method 300 can generally be performed by or under the control of the control unit 102.

With reference to Fig. 3, at block 302, contextual information associated with the infant is acquired. As mentioned earlier, the control unit 102 of the apparatus 100 can acquire contextual information associated with the infant from a memory 106 (which may be a memory 106 of the apparatus 100, an external memory 106 or both). Alternatively or in addition, the control unit 102 of the apparatus 100 can acquire contextual information associated with the infant by controlling at least one contextual information sensor 108 in the environment of the infant to acquire the contextual information.

At block 304, at least one heart rate variability signal is acquired from the infant. As mentioned earlier, a heart rate variability signal conveys (or represents) variations in a heart rate of the infant over time. In some embodiments, the at least one heart rate variability signal may be acquired from the infant simultaneously with (or at the same time as) the contextual information associated with the infant is acquired. As mentioned earlier, the control unit 102 of the apparatus 100 can acquire the at least one heart rate variability signal from the infant by controlling at least one heart rate variability sensor 104 to acquire the at least one heart rate variability signal from the infant.

At block 306, the at least one heart rate variability signal acquired from the infant is processed to determine a heart rate variability feature. In an example embodiment where the at least one heart rate variability signal is an electrocardiography (ECG) signal, the ECG signal may be processed to detect QRS complexes, to determine the intervals between adjacent QRS sequences and to determine a heart rate variability feature based on the determined intervals. In another example embodiment where the at least one heart rate variability signal is a photoplethysmography (PPG) signal or a ballistocardiography (BCG) signal, the PPG or BCG signal may be processed to determine intervals between consecutive pulse signals and to determine a heart rate variability feature based on the determined intervals. A determined heart rate variability feature according to any of the embodiments described herein can comprise any feature (or characteristic) of heart rate variability that can be determined from the at least one heart rate variability signal. For example, the determined heart rate variability feature can comprise time-domain features, frequency-domain features, non-linear features, or any other heart rate variability features, or any combinations of heart rate variability features.

Examples of the heart rate variability feature include, but are not limited to, an increase in heart rate variability signal, a decrease in heart rate variability signal, a standard deviation of a time interval between heart beats in the heart rate variability signal (or a standard deviation of NN-intervals, SDNN), a square root of mean squares of successive differences between adjacent heart beats in the heart rate variability signal (or a square root of the mean squares of the successive differences between adjacent NN-intervals, RMSSD), a number of interval differences of successive heart beat intervals in the heart rate variability signal that are greater than a threshold interval (or a number of pairs of NN-intervals that differ by more than the threshold interval, such as by more than 50 milliseconds, NN50), the proportion of interval differences of successive heart beat intervals in the heart rate variability signal that are greater than the threshold interval divided by the total number of heart beat intervals (such as the proportion of NN50 divided by the total number of NNs, pNN50), a power in the frequency domain of the heart rate variability signal (such as in the high frequency domain HF, the low frequency domain LF, or both), a normalized power in the frequency domain of the heart rate variability signal (such as the normalized power in the high frequency domain HF, the low frequency domain LF, or both), a cardiac autonomic balance of the heart rate variability signal (such as the ratio of the power in the low frequency domain LF and the power in the high frequency domain HF, i.e. LF/HF), a standard deviation perpendicular to a line of identity in a Poincaré plot of the heart rate variability signal SD1, a normalized SD1, a standard deviation along a line of identity in a Poincaré plot of the heart rate variability signal SD2, a normalized SD2, or any other heart rate variability feature, or any combination of heart rate variability features.

Although examples have been provided for the determined heart rate variability feature, it will be understood that any other heart rate variability feature, or any combinations of heart rate variability features, can be determined and processed in the manner described herein.

At block 308, the determined heart rate variability feature is assigned to a class according to the acquired contextual information. A class can be defined by one or more contextual information parameters. For example, one class may be for infants lying supine who are less than 36 weeks gestational age (wGA) and are in an active sleep (AS) state, while another class may be for infants who are feeding or having a diaper change. Although some examples of classes have been provided, it will be understood that a class may be defined by any number, and any combination, of contextual information parameters. A class may be assigned to the determined heart rate variability feature using any known classification technique. For example, the classification of the determined heart rate variability feature can involve logistic regression, a support vector machine, a convolutional neural network, or any other classification technique.

At block 310, the determined heart rate variability feature is compared to one or more corresponding heart rate variability features stored in the same class as the determined heart rate variability in a memory 106. A corresponding heart rate variability feature is a heart rate variability feature of the same type as the determined heart rate variability feature. Thus, for example, a determined standard deviation of NN-intervals is compared to a stored standard deviation of NN-intervals, and so on. As mentioned earlier, the one or more corresponding heart rate variability features may, for example, be stored in the memory 106 in the form of a histogram of infants, a class distribution of infants, and/or look-up table of infants. The histogram, class distribution, or look-up table of infants may be formed from infant population data. A stored heart rate variability feature is in the same class as the determined heart rate variability where the infant for which the heart rate variability feature is stored has at least some contextual information in common with the infant for which the heart rate variability is determined. For example, the infant for which the heart rate variability feature is stored may be in the same age group (such as pre-term, full-term, post-term, child, or similar) and be under one or more of the same influences (such as the same temperature change, the same intervention, the same feeding status, or similar) as the infant for which the heart rate variability is determined.

In some embodiments, the comparison of the determined heart rate variability feature with one or more corresponding heart rate variability features may comprise determining a ratio of the determined heart rate variability feature to one of the corresponding heart rate variability features stored in the same class as the determined heart rate variability in the memory 106. For example, for an infant under an ambient temperature T, a ratio may be determined of the heart rate variability feature determined for the infant at the temperature T to a corresponding infant population feature stored for the temperature T.

In any of the embodiments described herein, the method may further comprise plotting the determined heart rate variability feature against the acquired contextual information over time. In these embodiments, the comparison of the determined heart rate variability feature with one or more corresponding heart rate variability features may comprise comparing the plot of the determined heart rate variability feature against the acquired contextual information over time with a plot of the one or more corresponding heart rate variability features against corresponding contextual information over time. For example, where the determined heart rate variability feature is a standard deviation of NN-intervals (SDNN), the determined SDNN may be plotted against the ambient temperature in the environment of the infant. In this example, the determined SDNN versus ambient temperature plot may be compared to one or more corresponding SDNN versus ambient temperature plots that are stored in the same class as the determined SDNN in the memory 106. In another example, a heart rate variability feature may be plotted against an acquired respiration rate and an acquired overall heart rate for the infant and compared to one or more corresponding plots stored in the same class as the determined heart rate variability feature in the memory 106. In some embodiments, the current values may be divided by the corresponding population values stored in the memory 106.

At block 312, a health status of the infant is determined based on the comparison. The health status of the infant may, for example, be determined to be a normal (or regular) health status or an abnormal (or irregular) health status based on the comparison.

In some embodiments, an abnormal (or irregular) health status of the infant may be determined when the determined heart rate variability feature differs by more than a predefined threshold from the one or more corresponding heart rate variability features stored in the same class as the determined heart rate variability in the memory 106. The predefined threshold may be a value, such as an absolute value or a percentage value. An abnormal (or irregular) health status can be indicative of the infant being in pain, experiencing stress, experiencing discomfort, exhibiting signs of illness (for example, sepsis), or other negative factors. On the other hand, a normal health status of the infant may be determined when the determined heart rate variability feature differs by less than the predefined threshold from the one or more corresponding heart rate variability features stored in the same class as the determined heart rate variability in the memory 106.

In some embodiments, more specific details of the health status of the infant may also be determined. For example, where an abnormal (or irregular) health status of the infant may be determined, the determined heart rate variability feature may be used to assess a function of the infant concerned. For example, the heart rate variability feature of the LF/HF ratio for the infant may be used to assess a sympathetic/parasympathetic function for the infant.

In any of the embodiments disclosed herein, the method can further comprise controlling a user interface 112 to render (or output or provide) the determined heart rate variability feature, the determined health status of the infant, both the determined heart rate variability feature and the determined health status of the infant, or any other information, or any combination of information, resulting from the method disclosed herein. In some embodiments, the determined heart rate variability feature, the determined health status of the infant, or any other information, or any combination of information, resulting from the method disclosed herein, may be summarized or plotted over time to show progress or deterioration of the infant. As previously mentioned, the control unit 102 may be configured to control the user interface 112 to render (or output or provide) such information. In embodiments where an abnormal (or irregular) health status is determined for the infant, the method may further comprise controlling a user interface 112 to generate a notification (such as an alarm). The control unit 102 may be configured to control the user interface 112 to generate the notification. The notification can be provided to a user of the apparatus 100 such as a medical professional, a care taker, a family member, or any other user of the apparatus 100.

In any of the embodiments described herein, the method can further comprise selecting a baseline for the determined heart rate variability feature based on the acquired contextual information. For example, the at least one heart rate variability signal acquired from the infant may be plotted against the acquired contextual information over time and a time period in which the acquired contextual information is below a predefined threshold may be detected. This can be representative of a quiet time interval. In one example, the at least one heart rate variability signal acquired from the infant may be plotted against acquired motion information over time and a time period in which the acquired motion information is below a predefined threshold may be detected. This can be representative of a quiet time interval such as where movement originates from breathing or heart beat, rather than from limb movements. The baseline for the determined heart rate variability feature may be selected from corresponding heart rate variability features in the detected time period. In some embodiments, the baseline may be selected as a mean difference of heart rate variability features in the detected time period.

The determined heart rate variability feature can then be normalized with the selected baseline. For example, the determined heart rate variability feature may be normalized by dividing it by the baseline. This can stabilize the heart rate variability signal during noisy time periods. The normalization may be performed automatically on acquisition of a heart rate variability signal from an infant. In embodiments where the determined heart rate variability feature is normalized with a baseline, the comparison of the determined heart rate variability feature with one or more corresponding heart rate variability features (at block 310 of Fig. 3) may comprise comparing the normalized heart rate variability feature to one or more corresponding heart rate variability features stored in the same class as the normalized heart rate variability in a memory.

The contextual information used to normalize the heart rate variability in the manner described above can also be used to provide additional information about the health status of the infant. Thus, in the earlier example provided where motion information is used in the selection of the baseline for normalization, the motion information can also be used to provide additional information about the health status of the infant. For example, a pre-term infant usually sleeps 70% of 24h. A pre-term infant also moves during sleep, but not extensively. As such, if a pre-term infant is found from the motion information to be moving heavily (such as more than 10% in 24h), this can be a further indicator of an abnormal (or irregular) health status of the infant.

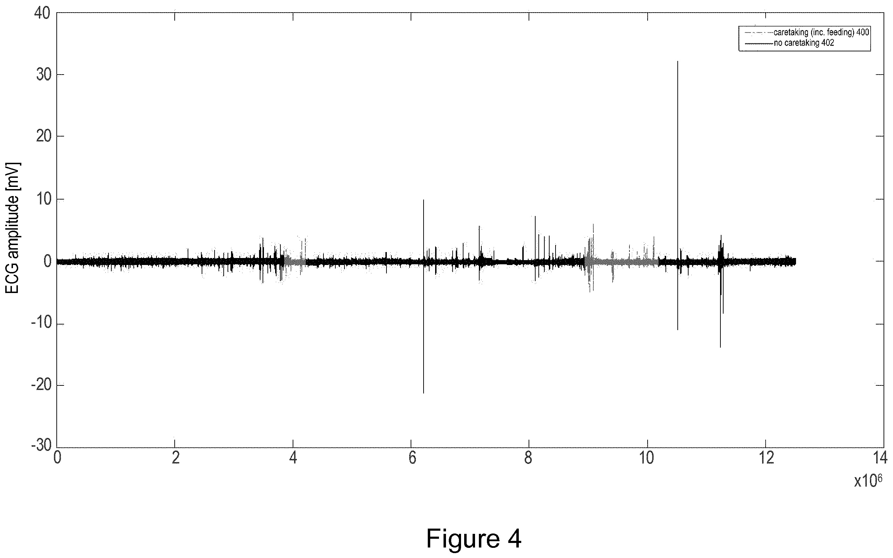

Fig. 4 is an illustration of an acquired electrocardiography (ECG) signal of an infant (specifically, a pre-term infant) according to an example embodiment. The ECG signal is representative of the heart rate variability of the infant and shows caretaking moments (such as feeding) of the infant.

In the same time period as the ECG signal is acquired (at block 302 of Fig. 3), contextual information associated with the infant is also acquired (at block 304 of Fig. 3). For example, a camera directed at the infant may be controlled to acquire contextual information indicative of the caretaking status of the infant (i.e. caretaking or no caretaking, which may include feeding or no feeding). The ECG signal is processed to determine a heart rate variability feature (at block 306 of Fig. 3). In the example embodiment of Fig. 4, the heart rate variability feature is the duration between R-peaks (the RR intervals). The RR intervals are assigned to a class (for example, "caretaking" 400 or "no caretaking" 402) according to the acquired contextual information associated with the infant (at block 308 of Fig. 3). There are two instances of "caretaking" 400 identified from the acquired contextual information, which can be seen in Fig. 4.

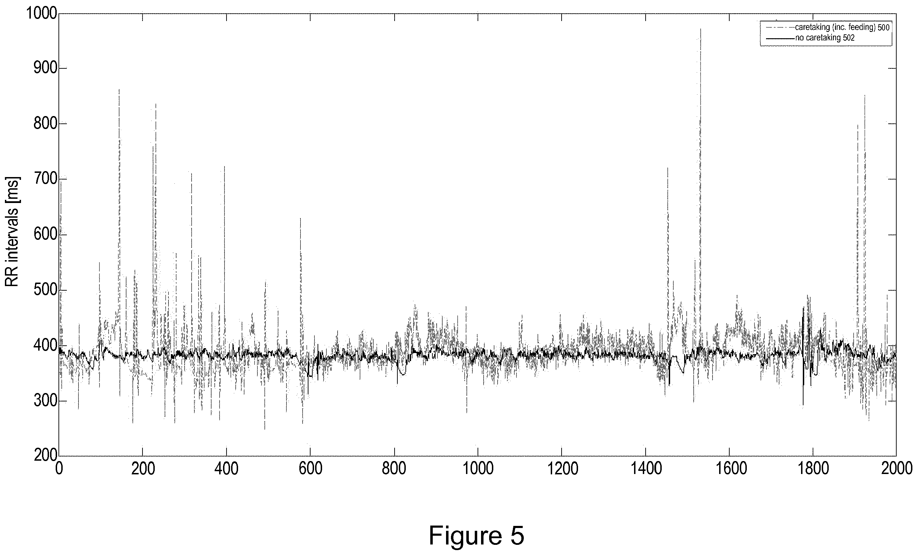

Fig. 5 is an illustration of the heart rate variability feature (the RR intervals) determined for the infant according to the example embodiment of Fig. 4. Specifically, Fig. 5 shows the determined RR intervals plotted on the y-axis versus the duration on the x-axis.

Fig. 5 can be used to compare the determined RR intervals during an identified "caretaking" instance 500 to the determined RR intervals during an identified "no caretaking" instance 502. Here, the identified "caretaking" instance 500 is representative of a known event (such as feeding) and the identified "no caretaking" instance 502 is representative of a detected stable (or quiet) time period for the infant (such as when there is no feeding). In Fig. 5, it can be seen that the standard deviation of the RR intervals are greater during the "caretaking" instance 500 than during the "no caretaking" instance 502.

The baseline for the RR intervals is selected by comparing the determined RR intervals during the identified "no caretaking" instance 502 (i.e. the stable time period) with the determined RR intervals during the identified "caretaking" instance 500. For example, the baseline for the RR intervals is selected by comparing the standard deviation of the determined RR intervals during the identified "no caretaking" instance 502 (i.e. the stable time period) with the standard deviations of the determined RR intervals during the identified "caretaking" instance 500.

The RR intervals are then normalized with the selected baseline. The normalized RR intervals are compared to one or more corresponding RR intervals stored in the same class as the normalized RR intervals in a memory (at block 310 of Fig. 3). A health status of the infant is then determined based on the comparison (at block 312 of Fig. 3). In some embodiments, the selected baseline can be used to train a machine learning algorithm to automatically determine the infant health status.

There is therefore provided an improved method and apparatus for determining a health status of an infant. According to method and apparatus described herein, it is possible to reliably determine a health status of an infant from heart rate variability features. The method and apparatus can be particularly useful in the neonatal domain, such as in a neonatal intensive care units (NICIs) or neonatal medium care units, or in the pediatric domain. The apparatus may be used in combination with existing patient (or infant) monitors.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (300) of operating an apparatus comprising a control unit to determine a health status of an infant, the method comprising:
acquiring (302) contextual information associated with the infant;
acquiring (304) at least one heart rate variability signal from the infant;
processing (306) the at least one heart rate variability signal acquired from the infant to determine a heart rate variability feature;
assigning (308) the determined heart rate variability feature to a class according to the acquired contextual information;
comparing (310) the determined heart rate variability feature to one or more corresponding heart rate variability features stored in the same class as the determined heart rate variability in a memory; and
determining (312) a health status of the infant based on the comparison.

2. A method as claimed in claim 1, wherein comparing comprises:
determining a ratio of the determined heart rate variability feature to one of the corresponding heart rate variability features stored in the same class as the determined heart rate variability in the memory.

3. A method as claimed in any one of the preceding claims, wherein determining a health status of the infant comprises:
determining an abnormal health status of the infant when the determined heart rate variability feature differs by more than a predefined threshold from the one or more corresponding heart rate variability features stored in the same class as the determined heart rate variability in the memory.

4. A method as claimed in any one of the preceding claims, the method further comprising:
plotting the determined heart rate variability feature against the acquired contextual information over time; and
wherein comparing comprises:
comparing the plot of the determined heart rate variability feature against the acquired contextual information over time with a plot of the one or more corresponding heart rate variability features against corresponding contextual information over time.

5. A method as claimed in any one of the preceding claims, the method further comprising:
selecting a baseline for the determined heart rate variability feature based on the acquired contextual information; and
normalizing the determined heart rate variability feature with the baseline.

6. A method as claimed in claim 5, wherein selecting a baseline comprises:
plotting the at least one heart rate variability signal acquired from the infant against the acquired contextual information over time;
detecting a time period in which the acquired contextual information is below a predefined threshold; and
selecting a baseline for the determined heart rate variability feature from corresponding heart rate variability features in the detected time period.

7. A method as claimed in claim 5 or 6, wherein comparing comprises:
comparing the normalized heart rate variability feature to one or more corresponding heart rate variability features stored in the same class as the normalized heart rate variability in a memory.

8. A method as claimed in any one of the preceding claims, wherein the determined heart rate variability feature comprises any one or more of:
an increase in heart rate variability signal;
a decrease in heart rate variability signal;
a standard deviation of a time interval between heart beats in the heart rate variability signal;
a square root of mean squares of successive differences between adjacent heart beats in the heart rate variability signal;
a number of interval differences of successive heart beat intervals in the heart rate variability signal that are greater than a threshold interval;
a power in the frequency domain of the heart rate variability signal;
a cardiac autonomic balance of the heart rate variability signal;
a standard deviation perpendicular to a line of identity in a Poincaré plot of the heart rate variability signal; and
a standard deviation along a line of identity in a Poincaré plot of the heart rate variability signal.

9. A method as claimed in any one of the preceding claims, wherein the contextual information comprises information that has an influence on the heart rate variability of the infant.

10. A method as claimed in any one of the preceding claims, wherein the contextual information comprises information on any one or more of:
an age of the infant;
a size of the infant;
a developmental stage of the infant;
a feeding status of the infant;
a sleep state of the infant;
a sleep or wake level of the infant;
a lying position of the infant;
a movement of the infant;
an activity level of the infant;
a heart rate of the infant;
a respiration rate of the infant;
a body temperature of the infant;
a skin color of the infant;
a facial expression of the infant;
an intervention with the infant;
an ambient temperature in the environment of the infant; and
an irregularity with equipment associated with the infant.

11. A computer program product comprising a computer readable medium, the computer readable medium having computer readable code embodied therein, the computer readable code being configured such that, on execution by a suitable computer or processor, the computer or processor is caused to perform the method of any one of claims 1-10.

12. An apparatus (100) for determining a health status of an infant, the apparatus (100) comprising:
a control unit (102) configured to:
acquire contextual information associated with the infant;
acquire at least one heart rate variability signal from the infant;
process the at least one heart rate variability signal acquired from the infant to determine a heart rate variability feature;
assign the determined heart rate variability feature to a class according to the acquired contextual information;
compare the determined heart rate variability feature to one or more corresponding heart rate variability features stored in the same class as the determined heart rate variability in a memory; and
determine a health status of the infant based on the comparison.

13. An (100) apparatus as claimed in claim 12, wherein the control unit (102) is configured to:
acquire the at least one heart rate variability signal from the infant by controlling at least one heart rate variability sensor (104) to acquire the at least one heart rate variability signal from the infant; and
acquire the contextual information associated with the infant from a memory (106) or by controlling at least one contextual information sensor (108) in the environment of the infant to acquire the contextual information.

14. An apparatus (100) as claimed in claim 13, wherein:
the at least one heart rate variability sensor (104) comprises any one or more of:
a camera;
a motion sensor;
a laser Doppler sensor;
a Doppler radar sensor;
a needle patch sensor;
an electrocardiography (ECG) sensor;
a photoplethysmography (PPG) sensor;
a ballistocardiography (BCG) sensor;
an instrumented catheter sensor; and
an instrumented feeding tube sensor,
the contextual information sensor (108) comprises any one or more of:
a camera directed at the infant;
an audio sensor in the environment of the infant;
a motion sensor in the environment of the infant;
a pressure sensor in a surface on which the infant lies;
a temperature sensor in the environment of the infant; and
a physiological characteristic sensor.

15. An apparatus (100) as claimed in any one of claims 12 to 14 wherein the control unit (102) is further configured to:
control a user interface (112) to output any one or more of the determined heart rate variability feature and the determined health status of the infant.
